# EUROPÄISCHE PATENTSCHRIFT

(11) **EP 1 971 683 B1**
(45) Veröffentlichungstag und Bekanntmachung des Hinweises auf die Patenterteilung: **04.01.2012**
(21) Anmeldenummer: 06828727.5
(22) Anmeldetag: 22.12.2006
(51) Int. Cl.: C12N 9/88, C12N 15/60, C12N 5/10

(54) **ADENYLAT-ZYKLASE, FÜR DIE ADENYLAT-ZYKLASE KODIERENDE GENSEQUENZ, VEKTOREN UND ZELLEN SOWIE DEREN VERWENDUNG**
ADENYLATE CYCLASE, GENE SEQUENCE CODING FOR ADENYLATE CYCLASE, VECTORS AND CELLS, AND THE USE THEREOF
ADENYLATE CYCLASE, SEQUENCE GENIQUE CODANT L'ADENYLATE CYCLASE, VECTEURS ET CELLULES ET LEUR UTILISATION

(30) Priorität: 07.01.2006 DE 102006000942
(43) Veröffentlichungstag der Anmeldung: 24.09.2008
(73) Patentinhaber: Forschungszentrum Jülich GmbH, 52425 Jülich (DE)
(72) Erfinder: BAUMANN, Arnd, 52428 Jülich (DE); WACHTEN, Sebastian, Cambridge, CB2 1 PD (GB)
(86) Internationale Anmeldenummer: PCT/DE2006/002314
(87) Internationale Veröffentlichungsnummer: WO 2007/079712

(56) Entgegenhaltungen:
- WACHTEN, SEBASTIAN: "Funktionelle Charakterisierung von Adenylatzyklasen der Honigbiene Apis mellifera" [Online] Dezember 2005 (2005-12), , XP002444846 Gefunden im Internet: URL:http://juwel.fz-juelich.de:8080/dspace /bitstream/2128/471/1/Juel_4196_Wachten.pd f> [gefunden am 2007-07-30] Frame 1, Nucleotides 814-3507 Seite 103 - Seite 105; Abbildung A1(2)
- WACHTEN SEBASTIAN ET AL: "Molecular identification and functional characterization of an adenylyl cyclase from the honeybee" JOURNAL OF NEUROCHEMISTRY, Bd. 96, Nr. 6, März 2006 (2006-03), Seiten 1580-1590, XP002444844 ISSN: 0022-3042
- LUDWIG M ET AL: "CHARACTERIZATION OF THE HUMAN ADENYLYL CYCLASE GENE FAMILY: cDNA GENE STRUCTURE, AND TISSUE DISTRIBUTION OF THE NINE ISOFORMS" JOURNAL OF RECEPTOR AND SIGNAL TRANSDUCTION, Bd. 22, Nr. 1-4, 2002, Seiten 79-110, XP002999990
- DATABASE EMBL [Online] 8. Dezember 2003 (2003-12-08), "Xenopus laevis type 7 adenylyl cyclase mRNA, partial cds." XP002444884 gefunden im EBI accession no. EMBL:AY462195 Database accession no. AY462195
- SEEBACHER T ET AL: "An isoform-specific interaction of the membrane anchors affects mammalian adenylyl cyclase type V activity." EUROPEAN JOURNAL OF BIOCHEMISTRY / FEBS JAN 2001, Bd. 268, Nr. 1, Januar 2001 (2001-01), Seiten 105-110, XP002444845 ISSN: 0014-2956
- HANOUNE J ET AL: "Regulation and role of adenylyl cyclase isoforms." ANNUAL REVIEW OF PHARMACOLOGY AND TOXICOLOGY 2001, Bd. 41, 2001, Seiten 145-174, XP002444859 ISSN: 0362-1642
- CANN M.; LEVIN L.: "Restricted expression of a truncated adenylyl cyclase in the cephalic furrow of Drosophila melanogaster", DEVELOPMENT GENES AND EVOLUTION, vol. 210, no. 1, January 2000 (2000-01), pages 34-40,

## Beschreibung

Die Erfindung betrifft eine Adenylat-Zyklase, eine für die Adenylat-Zyklase kodierende Gensequenz, Vektoren und Zellen sowie deren Verwendung.

Adenylat-Zyklasen synthetisieren den intrazellulären Botenstoff 3',5'-zyklisches Adenosin-Monophosphat (cAMP). Die Erhöhung der intrazellulären cAMP Konzentration ([cAMP]ᵢ) führt entweder über die direkte Bindung des cAMP an Zielproteine oder über cAMP-abhängige Phosphorylierung von Proteinen zu Änderungen zellulärer Signalprozesse. Neben einer löslichen Form wurden bisher ausnahmslos membranständige Adenylat-Zyklasen identifiziert und charakterisiert [Cooper, D.M.F (2003) "Regulation and organization of adenylyl cyclases and cAMP" Biochem. J. 375, 517-529]. Die Enzymaktivität der membranständigen Adenylat-Zyklasen wird durch stimulatorische oder inhibitorische, trimere GTP-bindende Proteine (Gₛ bzw. Gᵢ) reguliert, die wiederum durch G-Protein gekoppelte Rezeptoren (GPCR) aktiviert werden. Die Aktivität der Adenylat-Zyklasen kann des weiteren Ca²⁺-abhängig durch Calmodulin oder durch Phosphorylierung reguliert werden [Sunahara R.K. und Taussig, R, (2003) "Isoforms of mammalian adenylyl cyclase: multiplicities of signaling" Mol. Interv. 2,168-184].

In Wirbeltieren sind neun verschiedene Adenylat-Zyklasen bekannt, die nach ihren Aktivierungs- und Modulationseigenschaften klassifiziert werden. Eine ähnlich große Gruppe von Adenylat-Zyklasen ist für *Drosophila melanogaster* beschrieben [Cann, M.J., Chug. E, Levin. L.R. (2000) "A new family of adenylyl cyclase genes in the male germline of Drosophila melanogaster" Dev. Genes Evol. 210, 200-206; Iourgenko, V., Levin, L.R. (2000) "A calciuminhibited Drosophila adenylyl cyclase" Biochim. Biophys. Acta 1495, 125-139; Canu, M.J. Levin, L.R. (2000) "Restricted expression of a truncated adenylyl cyclase in the cephalic furrow of Drosophila melanogaster" Dev. Genes Evol. 210, 34-40; Levin, L.R., Han P-L., Hwang, P.M., Feinstein, P.G., Davis, R.L., Reed, R.R. (1992) "The Drosophila learning and memory gene rutabaga encodes a Ca2+ /calmodulin-responsive adenylyl cyclase" Cell 68, 479-489.]. Allerdings sind nur wenige dieser Enzyme biochemisch und pharmakologisch charakterisiert worden.

Die Veröffentlichung von SEEBACHER T ET AL: "An isoform- specific inteaction of the membrane anchors affects mammalian adenylyl cyclase type V activity" im European Journal of Biochemistry/ FEBS Jan 2001, Bd. 268, Nr. 1, Januar 2001 (2001-01), Seiten 105- 110, XP002444845 ISSN: 0014-2956 offenbart eine Adenylatcyclase, bei der der Sequenzabschnitt entsprechend M1 teilweise deletiert ist.

Die Veröffentlichung von CANN, M., LEVIN,L:"Restricted expression of a truncated adenylyl cyclase in the cephalic furrow of Drosophila melanogaster" in Development Genes and Evolution, Bd. 210, Nr. 1, Januar 2000 (2000-01), Seiten 34-40 offenbart eine Adenylatcyclase bei der der M1- Abschnitt komplett deletiert ist.

Die nach dem Stand der Technik bekannten membranständigen Adenylat-Zyklasen müssen stimuliert werden. Dies führt zu zeit-, material- und kostenintensiven Verfahrensweisen.

Es ist die Aufgabe der Erfindung, alternative Stoffe zur Verfügung zu stellen, mit denen die cAMP-Produktion gesteigert werden kann. Die cAMP-Produktion soll von Signalstoffen, deren Aktivität bzw. Konzentration entkoppelt werden. Es soll eine cAMP-abhängige Steigerung der Expression von unterschiedlichsten Genen, beispielsweise Reportergenen, ermöglicht werden. Die Aktivität von Ionenkanälen soll besser moduliert werden, beispielsweise soll die Permeabilität der Ionenkanäle gesteigert oder verringert werden. Es soll ein Test für Wirkstoffe (Screening) zur Verfügung gestellt werden, beispielsweise für G-Protein gekoppelte Rezeptoren, die die Adenylat-Zyklase-Aktivität regulieren. Es soll ein

Screening für Liganden ermöglicht werden, die inhibitorisch auf die Adenylat-Zyklase-Aktivität wirken. Es sollen Zellen, Proteine und DNA zur Verfügung gestellt werden, mit denen diese Vorteile bewirkt werden können bzw. die die gewünschten Eigenschaften, wie Steigerung der Aktivität der Adenylat-Zyklase gegenüber der Wildform, besitzen. Die Notwendigkeit einer Stimulation der Adenylat-Zyklase soll entfallen, beispielsweise durch Forskolin oder andere etablierte Adenylat-Zyklase-Aktivatoren.

Ausgehend vom Oberbegriff des Anspruchs 1 wird die Aufgabe erfindungsgemäß gelöst mit den Merkmalen des kennzeichnenden Teils des Anspruchs 1.

Mit den erfindungsgemäßen Stoffen ist die basale Aktivität der Adenylat-Zyklase gegenüber der Adenylat-Zyklase der Wildform um einen Faktor von ca. 4 bis 5 erhöht.

Die Aktivität ist entkoppelt von äußeren Signalstoffen. Gene, beispielsweise Reportergene, können cAMP-abhängig gesteigert exprimiert werden, was beispielsweise für die biotechnologische Produktion von Chemikalien, insbesondere Feinchemikalien vorteilhaft ist. Es können Screeningverfahren bereitgestellt werden, welche zeit-, material- und kostensparend sind und automatisiert werden können.

Vorteilhafte Weiterbildungen der Erfindung sind in den Unteransprüchen angegeben.

In den Zeichnungen sind verschiedene Erfindungsgegenstände schematisch dargestellt. Es zeigt:

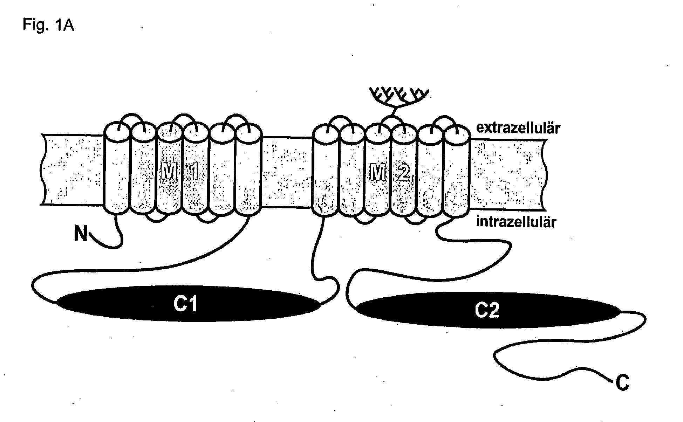

- Fig. 1A:: Eine Adenylat-Zyklase nach dem Stand der Technik.

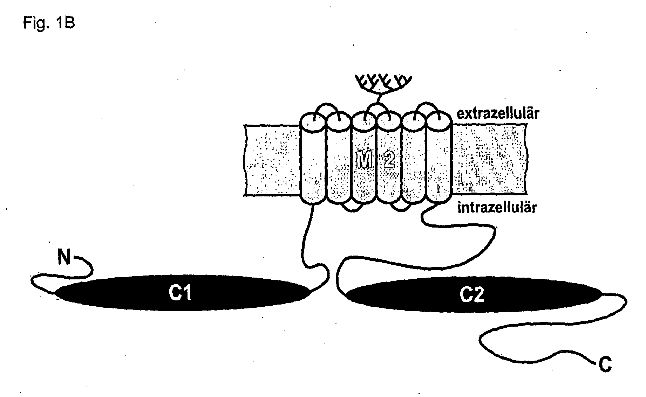

- Fig.1B:: Die erfindungsgemäß veränderte Adenylat-Zyklase.
- Fig.2:: Einen erfindungsgemäßen Vektor.

Im Folgenden soll die Erfindung erläutert werden.

Erfindungsgemäß wird eine membranständige Adenylat-Zyklase zur Verfügung gestellt, welche nur einen hydrophoben Sequenzabschnitt besitzt, der die Zellmembran beispielsweise sechs Mal durchspannt.

Erfindungsgemäß ist der erste hydrophobe Sequenzabschnitt und ggf. der natürlich vorkommende N-Terminus der Adenylat-Zyklase deletiert.

Die erfindungsgemäße Adenylat-Zyklase besitzt eine katalytische C1-Domäne, welche nicht mehr über eine N-terminale Fortsetzung der Aminosäuresequenz in die Zellmembran des Organismus, in dem sie sich befindet, eingelagert ist.

Die C1-Domäne der membranständigen Adenylat-Zyklase ist dabei funktionsfähig.

Die erfindungsgemäße Adenylat-Zyklase umfasst ein freies C-terminales und ein freies N-terminales Ende, welches in das Cytoplasma ragt. Das freie N-terminale Ende beinhaltet die C1-Domäne der Adenylat-Zyklase. Das C-terminale Ende beinhaltet die C2-Domäne der Adenylat-Zyklase.

Es ist aber möglich, dass sich am N-terminalen Ende der C1-Domäne noch Aminosäuren befinden, welche in die Membran inserieren. Dabei soll aber die Beweglichkeit der C1-Domäne im Cytoplasma gemäß dem Erfindungsgedanken nicht so eingeschränkt werden, dass die Aktivität der Adenylat-Zyklase gegenüber der oben genannten Deletionsmutante sehr verringert ist.

Erfindungsgemäß bevorzugt ist, dass die C1-Domäne der Adenylat-Zyklase frei in das Zellinnere ragt, ohne dass eine Fortsetzung der C1-Domäne an der Membran befestigt ist.

Es kann sich dabei um die erfindungsgemäß modifizierten Adenylat-Zyklase aus der Honigbiene handeln.

Die für die Proteinsequenzen möglichen Strukturen sind durch die Proteinsequenzen nach Seq. Nr. 2 gegeben.

Weiterhin ist auch die zu der Sequenz 2 zugehörige Nukleotidsequenz, insbesondere DNA-Sequenz nach der Sequenz Nr. 1 von der Erfindung umfasst.

In den Sequenzprotokollen sind einige der erfindungemäßen Proteine und der dafür kodierenden DNA-Sequenzen dargestellt.

Es zeigt:
- Seq. Nr. 1: DNA aus der Honigbiene, DSMZ Hinterlegungs-Nr. 17812, hinterlegt am 15.12.2005 (DSMZ-Deutsche Sammlung von Mikroorganismen und Zellkulturen GmbH, Mascheroder Weg 1b, D-38124 Braunschweig)
- Seq. Nr.2:: AC2T Protein aus der Honigbiene

So lange die Funktion der erfindungsgemäßen Adenylat-Zyklase nicht beeinträchtigt wird, kann deren Länge gegenüber den in den Sequenzprotokollen angegebenen Längen auch verkürzt sein. Hierbei können beispielhaft, aber nicht beschränkend, folgende Ausführungsformen genannt werden, die funktionsfähig sind, nicht die volle Länge besitzen, jedoch von der Erfindung umfasst sein sollen. Selbstverständlich können die Schnitte auch anders gelegt werden.

Die Sequenz des Proteins kann daher folgende Aminosäure umfassen:
- Seq.2:: 78-897

Die Sequenz der DNA kann folgende Nukleotide umfassen:
- Seq.1:: 1045-3507

Eine Übersicht der funktionellen Domänen der verschiedenen

Adenylat-Zyklasen ist in Tabelle 1 gegeben.

Bestandteil der Erfindung sind auch die DNA-Sequenzen einer 80%igen, 90%igen oder 95%igen Homologie.

Von der Erfindung sind auch die angegebenen Proteine einer 80%igen, 90%igen oder 95%igen Homologie umfasst.

Die Sequenzen der angegebenen Homologien können durch gerichtete oder ungerichtete Mutagenese erhalten werden.
Beispielsweise können Deletions-, Substitutions- oder Insertionsmutanten erzeugt werden.
Die dafür geeigneten Mittel sind UV-Strahlung, Röntgen-Strahlung, chemische Mutagene, Deletions-, Insertions- oder Substitutionsverfahren, wie beispielsweise gerichtete Mutagenese durch PCR, quick exchange Mutagenese Kits, Restriktion der DNA und Religation.

Die Erfindung betrifft auch Vektoren, welche die nach der Sequenz 1 angegebenen Struktur und deren Homologe bzw. verkürzte Formen beinhalten.

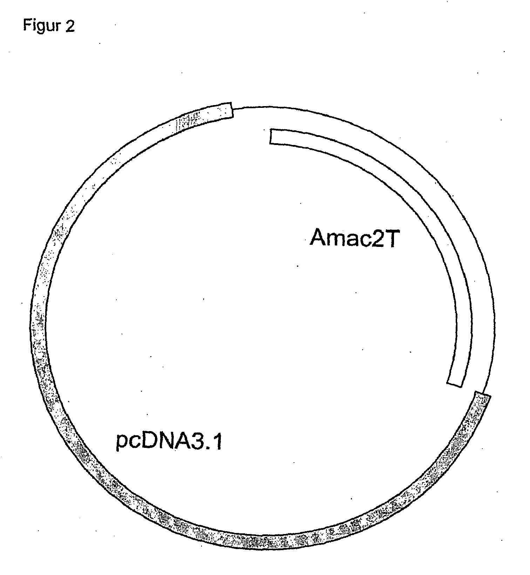

Als Vektoren können beispielsweise Plasmide oder Phagen dienen. Beispielhaft können p Bluescript, pc DNA, Zap II und Cosmide genannt werden. Der Vektor pc DNA mit der Sequenz Nr. 1 ist in Figur 2 dargestellt.

Die Erfindung betrifft auch Chromosomen, welche die erfindungsgemäßen Sequenzen enthalten. Die in die Chromosomen eingefügten DNA-Sequenzen erhöhen die endogene Aktivität der erfindungsgemäßen Adenylat-Zyklase.

Weiterhin sind von der Erfindung auch Zellen umfasst, die die erfindungsgemäße Adenylat-Zyklase, nach der Sequenz 2 oder deren Homologe und/oder die erfindungsgemäße Nukleotidsequenz 1 deren Homologe enthalten.

Typische Zellen sind humane Zellen, Zellen von der Maus, Ratte, Meerschwein, Affe, Vogel oder Insekten.

Bevorzugt sind dabei immortale Zellen.

Die Zelltypen sind beispielsweise, aber nicht beschränkend, humane embryonale Nierenzellen (HEK293), Cos-, ChO-, Nk2- Sf9- oder Hefezellen.

Letztlich können die erfindungsgemäßen Nukleotidsequenzen in jede Zelle eingebracht werden.

Die erfindungsgemäß veränderten Zellen produzieren cAMP unabhängig von extrazellulärer Stimulation. Durch die erfindungsgemäße Adenylat-Zyklase sowie die zugehörigen DNA-Sequenzen kann die cAMP-Produktion gegenüber der Wildform der Zelle gesteigert werden. So sind 4 bis 5-fache Steigerungen möglich.

Alle an die Bildung von cAMP gekoppelten Stoffwechelprozesse der Zelle können intrazellulär aber auch in vitro beschleunigt werden.

In Folge kann die Aktivität von Ionenkanälen modifiziert werden. Das cAMP kann direkt an den Ionenkanal binden. Die Kanäle öffnen und leiten einen Ionenstrom, was zu einem elektrischen Signal führt. Das cAMP aktiviert Proteinkinasen, die Phosphatgruppen auf die Ionenkanäle übertragen.

Durch die Phosphorylierung wird die Aktivität des Ionenkanals gesteigert, bzw. modifiziert.

Die Aktivität von Transkriptionsfaktoren kann gesteigert werden. Das cAMP aktiviert Proteinkinasen. Die Proteinkinasen übertragen Phosphatgruppen auf die Transkriptionsfaktoren. Auf diese Weise wird die Aktivität der Transkriptionsfaktoren gesteigert und Gene werden effizienter und effektiver abgelesen.

Es wird mehr cAMP zur Verfügung gestellt, welches an Zielproteine angelagert werden kann. Diese Proteine sind beispielsweise Ionenkanäle oder Proteinkinasen.

Die cAMP-abhängige Phosphorylierung von Proteinen kann gesteigert werden. Diese Proteine sind beispielsweise Ionenkanäle, Rezeptoren, Cytoskelettproteine oder Transkriptionsfaktoren. Hierbei wird die cAMP-Konzentration mittels der erfindungsgemäßen Adenylat-Zyklase erhöht.

Die Expression von Genen kann gesteigert werden, da viele Gene unter der Kontrolle cAMP-abhängiger Transkriptionsfaktoren stehen.

Mit der Steigerung der Expression der Gene kann auch die intrazelluläre Produktion von Chemikalien, insbesondere Feinchemikalien wie Aminosäuren, beschleunigt werden, indem die für den Biosyntheseweg der entsprechenden Verbindungen beteiligten Gene verstärkt exprimiert werden.

Reportergene können aktiviert werden. Durch die gesteigerte Aktivität von cAMP-abhängigen Transkriptionsfaktoren können Reportergene aktiviert werden, die über Bindestellen für diese Transkriptionsfaktoren verfügen.

Die Wirksamkeit von beispielsweise zyklisch Nukleotid-gesteuerten Ionenkanälen kann besser nachgewiesen werden, da die Ionenkanäle auf Grund der erhöhten cAMP-Konzentration geöffnet werden. Die Kanäle sind permeabel für Ca²⁺ Ionen. Der Anstieg der intrazellulären Ca²⁺ Konzentration kann mit Fluoreszenzfarbstoffen nachgewiesen werden und resultiert in erhöhten Fluoreszenzintensitäten.

Die Erfindung ermöglicht auch Verfahren zur Modulation der Ionenkanalaktivität, der Verstärkung der Expression von Genen, Verfahren zur Produktion von Chemikalien, beispielsweise Aminosäuren, Screening-Verfahren für Agonisten, Antagonisten von G-Protein gekoppelten Rezeptoren, welche von der erfindungsgemäßen Adenylat-Zyklase bzw. den zugehörigen DNA-Sequenzen Gebrauch machen.

Die erfindungsgemäßen Zellen können in einer bevorzugten Ausführungsform mit einem Gen eines cAMP-sensitiven Ionenkanals transfiziert sein. Vorzugsweise handelt es sich dabei um einen zyklisch Nukleotid-gesteuerten Ionenkanal.

In die erfindungsgemäß modifizierten Zellen können auch weitere Fremdgene transfiziert werden, die die cAMP-Konzentration beeinflussen. Diese können beispielsweise G-Protein gekoppelte Rezeptoren oder Phosphodiesterasen sein. Man kann somit testen, ob und wie effizient die zusätzlich exprimierten Proteine in den zellulären cAMP-Haushalt eingreifen. Damit werden die pharmakologischen Untersuchungen von G-Protein gekoppelten Rezeptoren, insbesondere von solchen Rezeptoren und den entsprechenden Signalwegen, die
Adenylat-Zyklasen inhibieren, verbessert.

### Beispiel:

Wir haben aus einer Kopf cDNA-Bibliothek von Honigbienen einen Klon isoliert, der für eine membranständige Adenylat-Zyklase (Seq.Nr.2) kodiert (AmAC2). Die Aminosäuresequenz der AmAC2 weist im Vergleich zu den bekannten membranständigen Adenylat-Zyklasen eine Besonderheit auf: Ein hydrophober Sequenzabschnitt, der die Zellmembran sechs Mal durchspannt, fehlt im AmAC2 Protein. Die Sequenzabschnitte, die für die Enzymaktivität benötigt werden, sind jedoch vollständig vorhanden.

Ziel der Erfindung ist es, die Nukleinsäure der Amac2 und Zelllinien, die die AmAC2 exprimieren, für pharmakologische Untersuchungen bereitzustellen. Unter Verwendung des Expressionsvektors pcDNA3.1(+) (Invitrogen, USA) haben wir das Adenylat-Zyklasegen (Amac2) stabil in das Genom von Zellen einer menschlichen embryonalen Nierenzelllinie (HEK-CNG-AC2) integriert. Diese Zelllinie ist zusätzlich mit dem Gen eines cAMP-sensitiven, zyklisch Nukleotid-gesteuerten Ionenkanals (CNG) stabil transfiziert. Der Ionenkanal kann als Biosensor für Änderungen der zellulären cAMP-Konzentration ([cAMP]ᵢ) eingesetzt werden, da er abhängig von der zellulären cAMP-Konzentration Kationen, wie z. Bsp. Natrium- oder Calcium-Ionen, in die Zelle einströmen lässt. Der Biosensor besitzt drei zentrale Funktionen:
1. Er "übersetzt" cAMP-Signale mit Hilfe von ionensensitiven Fluoreszenz-Indikatoren, wie z. Bsp. Fluo-4 direkt in lebenden Zellen in optische Signale.
2. Er verstärkt das biochemische Signal um mehrere Größenordnungen und verleiht dem Messsystem eine enorme Empfindlichkeit.
3. Er ermöglicht sowohl die Messung des Anstiegs als auch der Abnahme der [cAMP]ᵢ in stimulierten Zellen.

Die Änderung der intrazellulären Calcium-Konzentration kann mit Hilfe fluoreszierender oder lumineszierender Farbstoffe in so genannten Fluoreszenz- bzw. Lumineszenzreadern verfolgt und aufgezeichnet werden. Die Amplitude und die Geschwindigkeit, mit der sich die Fluoreszenz ändert, korrelieren dabei mit der [cAMP]ᵢ.

Wir haben gefunden, dass HEK-CNG-AC2 Zellen eine deutlich höhere basale [cAMP]ᵢ besitzen als die Zellen, die das AmAC2-Protein nicht exprimieren (~8 pmol cAMP/mg Protein in HEK-CNG-AC2 Zellen; 2,3 pmol cAMP/mg Protein in HEK-CNG Zellen). Inkubiert man die Zellen mit dem Phosphodiesterase Inhibitor IBMX, so steigen die Werte auf 43,6 pmol cAMP/mg Protein in der HEK-CNG-AC2 Zelllinie und auf -9 pmol cAMP/mg Protein in der HEK-CNG Zelllinie an. Durch den extrazellulären Botenstoff Noradrenalin, der endogene β-adrenerge Rezeptoren aktiviert, kann die AmAC2 stimuliert werden. Bei Verwendung von 30 µM Noradrenalin steigt die [cAMP]ᵢ in HEK-CNG-AC2 Zellen auf -6.800 pmol cAMP/mg Protein und in HEK-CNG Zellen auf ~166 pmol cAMP/mg Protein.

In die HEK-CNG-AC2 Zelllinie könnten weitere Fremdgene transfiziert werden, die die [cAMP]ᵢ beeinflussen. Man kann somit testen, ob und wie effizient die zusätzlich exprimierten Proteine in den zellulären cAMP-Haushalt eingreifen. Die Erfindung erleichtert prinzipiell die pharmakologische Untersuchung von G-Protein gekoppelten Rezeptoren, insbesondere von solchen Rezeptoren und den entsprechenden Signalwegen, die Adenylat-Zyklasen inhibieren. Für die Analyse solcher Rezeptoren war es bislang notwendig, zunächst die zelluläre cAMP-Synthese zu stimulieren und damit die [cAMP]ᵢ Zu erhöhen. Da die HEK-CNG-AC2 Zelllinie bereits eine deutlich erhöhte [cAMP]ᵢ aufweist, kann dieser Arbeitsschritt entfallen, der ansonsten einen zusätzlichen Aufwand an Zeit und Arbeitsschritten erfordert. Die erforderliche Messdauer sinkt dadurch um etwa 50 % und gleichermaßen steigen die Geschwindigkeit und der Durchsatz bei pharmakologischen Untersuchungen. Die HEK-CNG-AC2 Zelllinie kann somit die Effizienz bei der pharmazeutischen Wirkstoffsuche wesentlich steigern.

Die Erfindung betrifft insbesondere
1. Nukleinsäuresequenz der Adenylat-Zyklase Subtyp2 aus der Honigbiene (Amac2) und das entsprechende Protein (AmAC2).
2. Nukleinsäuresequenzen, die Sequenzähnlichkeiten mit dem Amac2-Gen gemäß Punkt 1 besitzen (Homologe) und denen insbesondere der transmembranale Sequenzabschnitt fehlt, der für andere membranständige Adenylat-Zyklasen typisch ist.
3. Proteine, die den Nukleinsäuresequenzen gemäß der Punkte 1 und 2 entsprechen und ähnliche funktionelle Eigenschaften, insbesondere eine hohe basale Aktivität besitzen wie die AmAC2.
4. Verwendung der Nukleinsäuresequenzen gemäß der Punkte 1 und 2 und der Proteine gemäß Punkt 3 für pharmakologische Assays.
5. Verwendung von Assays gemäß Punkt 4, die mit radioaktiven, immunologischen, Fluoreszenz- oder Lumineszenzbasierten Methoden Änderungen der zellulären cAMP-Konzentration nachweisen.
6. Verwendung von Assays gemäß Punkt 5, die einen zyklisch Nukleotid-gesteuerten Ionenkanal als Biosensor für Änderungen der zellulären cAMP-Konzentration verwenden.
7. Verwendung von Assays gemäß Punkt 5 mit dem Ziel, pharmakologische Wirkstoffe zu finden, die die Aktivität von Adenylat-Zyklasen gemäß Punkt 3 regulieren oder modulieren.
8. Verwendung von Assays gemäß Punkt 5 für die pharmakologische Charakterisierung von G Protein-gekoppelten Rezeptoren und den entsprechenden Signalwegen, insbesondere für G Protein-gekoppelte Rezeptoren, die Adenylat-Zyklasen inhibieren.

### Assaydurchführung

Die Zellen, die für die Versuchsdurchführung benutzt werden, sind stabil mit dem Gen des zyklisch Nukleotid-gesteuerten Ionenkanals (CNG) und dem Gen der Amac2t transfiziert. Die Zellen exprimieren konstitutiv sowohl das CNG-Kanalprotein als auch das AmAC2T-Protein. In die Zellen können weitere Gene transfiziert werden, die beispielsweise für G Protein-gekoppelte Rezeptoren kodieren. Um heraus zu finden, ob und wie diese Rezeptoren auf die Adenylat-Zyklase-Aktivität wirken, müssen die Zellen wie folgt behandelt werden:
Das Nährmedium wird gegen eine "Beladelösung" ausgetauscht, die einen Ca²⁺-sensitiven Fluoreszenzfarbstoff (beispielsweise Fluo-4) und optional den Phosphodiesterase-Inhibitor IBMX in gepufferter Lösung enthält. Der Fluoreszenzfarbstoff diffundiert in die Zelle(n) und reichert sich dort an. Wird der Rezeptor (s. o.) aktiviert und stimuliert dieser die Adenylat-Zyklase, so steigt die intrazelluläre cAMP-Konzentration. Das cAMP bindet an den CNG-Kanal. Der Kanal öffnet und Ca²⁺-Ionen fließen in die Zelle. Die Ca²⁺-Ionen binden an den Fluo-4 Farbstoff. Wird der Farbstoff mit Licht der Wellenlänge (λ = 480 nm) angeregt, so emittiert er bei ~ 520 nm Fluoreszenzlicht. Die Lichtemission des Farbstoffs ist umso größer, wenn er Ca²⁺-Ionen gebunden hat. Das bedeutet für das o. a. Beispiel, dass über die Rezeptoraktivierung mehr cAMP produziert wird und folglich mehr Ca²⁺-Ionen in die Zelle fließen. Vom Fluo-4 wird deshalb mehr Fluoreszenzlicht emittiert. Das Fluoreszenzsignal ist also direkt mit dem "Rezeptorsignal" gekoppelt.
   Führt hingegen die Rezeptoraktivierung zu einer Inhibierung der Adenylat-Zyklase, so sinkt die intrazelluläre cAMP-Konzentration. Infolgedessen öffnen weniger CNG-Kanäle und weniger Ca²⁺-Ionen fließen in die Zelle. Die Konsequenz ist eine Abnahme des vom Fluo-4 emittierten Fluoreszenzlichts. Dieser Reaktionsweg kann in Anwesenheit von IBMX verfolgt werden. Da die AmAC2T eine gesteigerte basale Enzymaktivität besitzt, kann die Enzyminhibierung infolge der Rezeptoraktivierung als reduzierte Fluoreszenzintensität nachgewiesen werden.
   Für die Messung des emittierten Fluoreszenzlichts können Fluoreszenz- und/oder Lumineszenzreader eingesetzt werden. Die Rohdaten werden digitalisiert und ausgewertet.

Die Funktion der offenbarten Sequenzen, insbesondere der humanen Sequenzen ist, dass sie für Adenylat-Zyklasen kodieren, welche zu einer gesteigerten cAMP-Produktion führen.
Es kodiert Seq. Nr. 1 für das Protein nach Seq. Nr.2.

Die Verwendung für die gewerbliche Anwendbarkeit liegt für die DNA-Sequenz und für das Protein in der Steigerung der c-AMP-Produktion.

In der Medizin nehmen G-Protein gekoppelte Rezeptoren (GPCR) eine Schlüsselposition ein. Etwa 40 % aller verschreibungspflichtigen Medikamente, die derzeit auf dem Markt sind, wirken auf GPCR. Unter diesen Medikamenten befinden sich unter anderem Betablocker, Neuroleptika, Antihistaminika, Opioide und Sympathomimetika. Neue Substanzklassen, wie beispielsweise die Triptane, Setrone und Sartane, die ebenfalls über GPCR wirken, haben sich in den letzten Jahren sowohl therapeutisch als auch kommerziell als erfolgreich erwiesen.

Adenylat-Zyklasen sind bedeutende Zielenzyme GPCR gesteuerter Signalketten. Zelllinien, die die AmAC2 exprimieren, eignen sich daher für pharmakologische Untersuchungen von Wirkstoffen, die GPCR aktivieren bzw. inhibieren. Mit der bereits o. g. Biosensor Zelllinie (s. S. 11-13 des Antrags), die neben dem AmAC2 Protein auch einen cAMP-sensitiven, zyklisch Nukleotid-gesteuerten Ionenkanal (CNG) exprimiert, können Änderungen der zellulären cAMP Konzentration ([cAMP]ᵢ) unmittelbar in lebenden Zellen verfolgt werden. Der CNG Kanal leitet abhängig von der zellulären [cAMP]ᵢ Kationen, wie z. Bsp. Natrium- oder Calcium-Ionen, in die Zelle. Der Ioneneinstrom kann über ionensensitive Fluoreszenzfarbstoffe gemessen und quantifiziert werden. Der Biosensor hat mehrere zentrale Funktionen:
1. Er "übersetzt" cAMP-Signale mit Hilfe von ionensensitiven Fluoreszenz-Indikatoren, wie z. Bsp. Fluo-4, direkt in lebenden Zellen in optische Signale.
2. Er verstärkt das biochemische Signal um mehrere Größenordnungen und verleiht dem Meßsystem eine enorme Empfindlichkeit.
3. Er ermöglicht sowohl die Messung des Anstiegs als auch der Abnahme der [cAMP]ᵢ in stimulierten Zellen.

Die Änderung der intrazellulären Calcium Konzentration kann mit Hilfe fluoreszierender oder lumineszierender Farbstoffe in Fluoreszenz- bzw. Lumineszenzreadern verfolgt und aufgezeichnet werden. Die Amplitude und die Geschwindigkeit, mit der sich die Fluoreszenz ändert, korrelieren dabei mit der [cAMP]ᵢ.
In die Biosensor Zelllinie können z. Bsp. GPCR-kodierende Gene transfiziert werden. Die pharmakologische Wirksamkeit neuer oder bereits bekannter Substanzklassen kann dann über Änderungen der [cAMP]ᵢ bestimmt werden. Für die Analyse solcher Rezeptoren, die die Enzymaktivität der Adenylat-Zyklase erniedrigen, war es bislang notwendig, zunächst die zelluläre cAMP-Synthese zu stimulieren und damit die [cAMP]ᵢ zu erhöhen. Da die HEK-CNG-AC2 Zelllinie bereits eine deutlich erhöhte [cAMP]ᵢ aufweist, kann dieser Schritt entfallen, der ansonsten einen zusätzlichen Aufwand an Zeit und Arbeit erfordert. Die experimentelle Messdauer sinkt dadurch um etwa 50 % und gleichermaßen steigen die Geschwindigkeit und der Durchsatz bei pharmakologischen Untersuchungen.

**Tabelle 1:**

| Übersicht der funktionellen Domänen der verschiedenen Adenylat-Zyklasen | | | |
|---|---|---|---|
| Bezeichnung | C1-Domäne | M2-Domäne | C2-Domäne |
| | | | |
| Seq.2, Honigbiene | 79-210 | 404-633 | 692-834 |

Die Ziffern geben die Aminosäurepositionen der einzelnen Bereiche entsprechend der Sequenzprotokolle an.

### Organization Applicant

Street :
City : Jülich
State :

Country :

Postalcode : 52425
PhoneNumber :

FaxNumber :

EmailAddress :
<110> OrganizationName : Forschungszentrum Jülich GmbH

### Application Project

<120> Title : Adenylat-Zyklase, für die Adenylat-Zyklase kodierende Gensequenzen, Vektoren und Zellen sowie deren verwendung
<130> AppFileReference : PT1.2259
<140> CurrentAppNumber :
<141> CurrentFilingDate :

### Sequence

<213> OrgamsmName : Honigbiene
<400> Presequencestring : 
ggcacgagtt cacgttagcg agcggctacc atgcacgcgt cgatttagca tcagacagcc 60
ggtaactggc ggagatacgc gcgcgttctc tgctagaaac tccgacgttt cttcaaatca 120
tcaatgaaac agtgatcatt gtcgtttatc ttgttcatca cgtgaggrtt atatgagacc 180
attgtttgca tattatatat ttagtccatt cgacgagatt taaaattatt caaacggatt 240
gtttgtcgtt tagagagtgg aattgaagga agaggaagaa ggtattttcg gaatcgaaga 300
cacaattcaa atacaaaaac ctagagaagc tgttcacggt ttaccaaagg aggattcagt 360
atggttatct ctcgcttttt gtcctcttgc aattggccct cggattcacg tattgcttga 420
ttctgataac aatggtcaac attgagaact gcgtaccgga tgtggtgatc agcacattag 480
tgagcgcttt attctgtcca gtgattgctc tttcgttcaa gtcaaagatc attattagaa 540
acacttacct atcggtcata ctatcctgtc tgatcgtggt attattagca attggggact 600
tggctgtccc cttctattac actttgtaca acgatgaagc accaccgata aggcctgctt 660
acgcgactca cgcgctcttg gcatgctatg tcttcctacc tctcacgaaa tatttacatg 720
ctctcatact cgggattacg actactacgt gctacttagt cgctttatcg ttaatcactt 780
atagaaattc attcgatttt agcacaaaga ttcatgaatg aaattgttac aagacgatca 840
tttctcgacc atcgaaaatg cgtggagtcg acgttgcggt tgaattatga gaaagatcaa 900
gaagaacaac ttacgcgaag tatattaccg caacatatag ctgcgaagat aaagaaagaa 960
tttagagata tatttaaatt tttagaagaa cacaaaaaac ctcccaaacc atctagtgat 1020
ttgtgcgtcg aaacgcacaa taatgtaagc atttgtatg cggatgttgt gaatttttct 1080
ggattgaccg tgacgttgcc agttcgaaaa ttggtggaga cattgaacga tttgtttggc 1140
agtttcgacg aagcttccga aaggcacaat gttctgcgta taaaattttt gggcgattgt 1200
tattattgtg taagcggtgt gccgacacca aattctcaac atgcgaaaag ctgcgtggat 1260
ttaggactcg atatgataaa gataattaaa gacgtaaggg cgaaagtgcg acgggagagc 1320
 
ggtgtggatg taaatatgag gataggtgtt cactcgggca atataatatc aggaattcta 1380
ggtgtgagca aatggcagta cgatgtttgg tcacgcgatg ttgttatagc taataaaatg 1440
gagcaaacag ggaaacccgg caaagttcat attacgcagc aaacgttgga tctcgtggat 1500
gctaacgaat atgattgcgt tccggtggat tctttgaacg atgaggttct caaaaagtat 1560
gggattcgaa gttatcttat tacaccttcg atacccgatc ccgattacga attcgaatct 1620
ccgactatgc agaacagcga gaacaccttg acagtcatga ctccgtcccc tgaaacctct 1680
aatacgcatt acgtgaaatt ttatgtacga tccagtacca gtatgaatcc cagctcgagg 1740
aacagtagac gacttaccgc cactataaat aatcatgttg gggtattcac cagcacgtgt 1800
agacgacgta cacattttat ggattcctgt ttgcgagatt atcatctaat gcttaaaact 1860
gcggatgcag aaatggaaca tgctattaag aaaatgcctt taactaaatg gaaacaatgg 1920
tgcaattgga aggatatgaa tccactattt ttaacatttc aacaatggag ttgggaaata 1980
ccgtttttaa agcaacctga tccacttttt aaattttata tcagttgtgc tctttttgtc 2040
ttaattttta tgggactcat ttatctggta cctacgcttt ctctttccaa atggcatccg 2100
agcacaacga ttagttattc tgcagcaatg ttatttttaa tcgctttaat gccaatagct 2160
tggatgcatt tcgcatggaa ccgatataaa gatccgcatg atgaacatga aggacagatt 2220
cctcatcctc gaaataaatt attatgtttt ttatatcaaa ctagcataaa ggttgtttgg 2280
agtgcatttc tcaggacagt tttatatttg gtaataacgt taatgttagc agcgtgtgcc 2340
atgtttgata tgatttttga atgtaagaat gttaatgagc ttacaagtaa taatataaca 2400
ttcagcgtaa tagaacctgg cacttcgaaa cttgattgca tagctacacc ttggcaaatg 2460
acgcaaactt gctccttggc tattttgacg agttttctgt tttaagactc ccattatatc 2520
ttaaaattag taataggcat cgtaatagtc gtattttatt catggaatgt ttggatatat 2580
cgttcaaatt tatttcagtc aggcgacaga tggaatccat atttagaacc aagactggct 2640
cacattttaa gcatagtttt tcttactttt tctttgcatc tgatagatcg tcaggcagaa 2700
tatttgaatc gattagatta cttatggaaa cgtcaattga ccaaagaaca agatgaagca 2760
ttccatacaa gaaatgctaa taaattttta cttcgtaata ttttaccaga acatgttgcg 2820
gagttctact tgaatatgaa tagaacagta gaaaatgagc cctatcatga agctcatagt 2880
aacgtcgctg taatgtttgc ttcattaacg gatgtatcca tcgatgaaag caatacttta 2940
gcagtcttaa atgagattat atgcgaattt gacaaattgc tatttgagcc atatttcaat 3000
cgaattgaaa aaattaaaat tgctggcact acttatatgg cagcttgtgg attagaagct 3060
agtcgacgcg attcaatgcg tagcccagaa aacgaggaaa atattactga taatgtggta 3120
aaagtgatgg cccaatttgc agtcgaaatg atagcagttc tagataaatt gaaaatgaga 3180
acctttacaa catccaaacc ttacagattg agaattggaa tatcacatgg tgaagttaca 3240
gcaggagtag taggtgctca aaaaccgtta tatgatatct ggggcgatgc tgtgaacatg 3300
gcgtcgagaa tggatacaac aggtgttcca ggaaaaatac agattacagc agaaactgcg 3360
 
gctgttcttg aacaacaagg tatcaagtgc catcttcgag gagatacgta tgtcaaacca 3420
aaaggtttag tcacaacgta ttttattggt atcgacgata atcgacagct tcaaagaaca 3480
aattctatcg aagaaaccag tctgtgatgg aaatactaaa ttgatcttta atcaattatt 3540
tcatttcatc gtaaatcgaa ttgactggaa gattatgtga aaaataagat aataaatatc 3600
taaatgaacg cacagagaga taggaaaaaa ccaaagaaaa tctaaaaaac caaaaaaaaa 3660
aaaaaaaaaa cc 3672
<212> Type : DNA
<211> Length : 3672
   SequenceName : EM2487 seq 1 SequenceDescription :

### Custom Codon

Sequence Name : EM2487 seq 1

### Sequence

<213> OrgamsmName : Honigbiene
<400> PreSequenceString : 
MNEIVTRRSF LDHRKCVEST LRLNYEKDQE EQLTRSILPQ HIAAKIKKEF RDIFKFLEEH 60
KKPPKPSSDL CVETHNNVSI LYADVVNFSG LTVTLPVRKL VETLNDLFGS FDEASERHNV 120
LRIKFLGDCY YCVSGVPTPN SQHAKSCVDL GLDMIKIIKD VRAKVRRESG VDVNMRIGVH 180
SGNIISGILG VSKWQYDVWS RDVVIANKME QTGKPGKVHI TQQTLDLVDA NEYDCVPVDS 240
LNDEVLKKYG IRSYLITPSI PDPDYEFESP TMQNSENTLT VMTPSPETSN THYVKFYVRS 300
STSMNPSSRN SRRLTATINN HVGVFTSTCR RRTHFMDSCL RDYHLMLKTA DAEMEHAIKK 360
MPLTKWKQWC NWKDMNPLFL TFQQWSWEIP FLKQPDPLFK FYISCALFVL IFMGLIYLVP 420
TLSLSKWHPS TTISYSAAML FLIALMPIAW MHFAWNRYKD PHDEHEGQIP HPRNKLLCFL 480
YQTSIKVVWS AFLRTVLYLV ITLMLAACAM FDMIFECKNV NELTSNNITF SVIEPGTSKL 540
DCIATPWQMT QTCSLAILTS FLFLRLHYIL KLVIGIVIVV FYSWNVWIYR SNLFQSGDRW 600
NPYLEPRLAH ILSIVFLTFS LHLIDRQAEY LNRLDYLWKR QLTKEQDEAF HTRNANKFLL 660
RNILPEHVAE FYLNMNRTVE NEPYHEAHSN VAVMFASLTD VSIDESNTLA VLNEIICEFD 720
KLLFEPYFNR IEKIKIAGTT YMAACGLEAS RRDSMRSPEN EENITDNVVK VMAQFAVEMI 780
AVLDKLKMRT FYTSKPYRLR IGISHGEVTA GVVGAQKPLY DIWGDAVNMA SRMDTTGVPG 840
KIQITAETAA VLEQQGIKCH LRGDTYVKPK GLVTTYFIGI DDNRQLQRTN SIEETSL 897
<212> Type : PRT
<211> Length : 897
   SequenceName : EM2487 seq 2
   SequenceDescription :

## Patentansprüche

1. Adenylat-Zyklase umfassend eine C1- und eine C2-Domäne,
**dadurch gekennzeichnet,**
**dass** sie eine Aminosäurefolge einer 80 %igen Homologie zu der Sequenz Nr. 2 besitzt und dass sie einen hydrophoben Sequenzabschnitt besitzt, welcher befähigt ist, sich in die sie aufnehmende Membran einzulagern und dass der erste hydrophobe Sequenzabschnitt (M1) der Adenylat-Zyklase deletiert ist.

2. Adenylat-Zyklase nach Anspruch 1,
**dadurch gekennzeichnet, dass** sie eine Aminosäuresequenz nach der Sequenz Nr. 2 besitzt.

3. Nukleinsäure, kodierend für eine Adenylat-Zyklase,
**dadurch gekennzeichnet,**
**dass** sie eine 80 %ige Homologie zu der Nukleinsäure gemäß Seq. Nr. 1 besitzt.

4. Nukleinsäure nach Anspruch 3,
**dadurch gekennzeichnet,**
**dass** sie eine Sequenz gemäß Seq. Nr. 1 besitzt.

5. Vektor enthaltend eine Nukleinsäure nach einem der Ansprüche 3 oder 4.

6. Zelle mit einem Vektor nach Anspruch 5,
**dadurch gekennzeichnet,**
**dass** sie eine Adenylat-Zyklase nach einem der Ansprüche 1 oder 2 enthält.

7. Zelle mit einem Vektor nach Anspruch 5,
**dadurch gekennzeichnet,**
**dass** sie eine Nukleinsäure nach einem der Ansprüche 3 oder 4 enthält.

## Claims

1. Adenylate cyclase comprising a C1 and a C2 domain,
**characterised in that**,
it has an amino acid sequence 80% homologous with sequence No. 2 and that it has a hydrophobic sequence section, which is capable of being deposited in the membrane accepting it and that the first hydrophobic sequence section (M1) of the adenylate cyclase is deleted.

2. Adenylate cyclase according to claim 1,
**characterised in that** it has an amino acid sequence according to sequence No. 2.

3. Nucleic acid, coding for an adenylate cyclase,
**characterised in that**,
it is 80% homologous with the nucleic acid according to sequence No. 1.

4. Nucleic acid according to claim 3,
**characterised in that**,
it has a sequence according to sequence No. 1.

5. Vector containing a nucleic acid according to one of claims 3 or 4.

6. Cell with a vector according to claim 5,
**characterised in that**,
it contains an adenylate cyclase according to one of claims 1 or 2.

7. Cell with a vector according to claim 5,
**characterised in that**,
it contains a nucleic acid according to one of claims 3 or 4.

## Revendications

1. Adénylate cyclase comprenant un domaine C1 et un domaine C2, **caractérisée en ce**
**qu'**elle possède une séquence d'acides aminés présentant une homologie de 80 % par rapport à la séquence n° 2 et en ce qu'elle possède un segment de séquence hydrophobe qui est capable de s'insérer dans la membrane réceptrice, et en ce que le premier segment de séquence hydrophobe (M1) est supprimé de l'adénylate cyclase.

2. Adénylate cyclase selon la revendication 1,
**caractérisée en ce qu'**elle possède une séquence d'acides aminés selon la séquence n° 2.

3. Acide nucléique codant pour une adénylate cyclase,
**caractérisé en ce**
**qu'**il possède une homologie de 80 % par rapport à l'acide nucléique selon SEQ ID N° : 1.

4. Acide nucléique selon la revendication 3,
**caractérisé en ce**
**qu'**il possède une séquence selon SEQ ID N° : 1.

5. Vecteur contenant un acide nucléique selon l'une des revendications 3 ou 4.

6. Cellule comportant un vecteur selon la revendication 5,
**caractérisée en ce**
**qu'**elle contient une adénylate cyclase selon l'une des revendications 1 ou 2.

7. Cellule comportant un vecteur selon la revendication 5,
**caractérisée en ce**
**qu'**elle contient un acide nucléique selon l'une des revendications 3 ou 4.
